# EUROPEAN PATENT SPECIFICATION

(11) **EP 2 683 424 B1**
(45) Date of publication and mention of the grant of the patent: **08.07.2015**
(21) Application number: 12710352.1
(22) Date of filing: 07.03.2012
(51) Int. Cl.: A61M 3/02

(54) **AN APPARATUS FOR PERFORMING TRANSANAL IRRIGATIONS**
VORRICHTUNG ZUR DURCHFÜHRUNG VON TRANSANALEN SPÜLUNGEN
APPAREIL DE RÉALISATION D'IRRIGATIONS TRANSANALES

(30) Priority: 10.03.2011 IT BO20110116
(43) Date of publication of application: 15.01.2014
(73) Proprietor: Di Martino, Renato, 40068 San Lazzaro Di Savena (BO) (IT)
(72) Inventor: Di Martino, Renato, 40068 San Lazzaro Di Savena (BO) (IT)
(74) Representative: Ruzzu, Giammario
(86) International application number: PCT/IB2012/051060
(87) International publication number: WO 2012/120456

(56) References cited:
- WO-A1-01/08614
- WO-A1-2004/006993
- WO-A2-98/20722
- CA-A1- 2 002 683
- US-A- 5 199 945

## Description

### Technical Field

The present invention relates to the technical field concerning production of medical devices and equipments.

In particular, the invention relates to an apparatus capable of carrying out, in automatic or semi-automatic way, intestinal irrigation operations intended to help evacuation of faeces, mainly in subjects with limited or absent control of the bowel functions.

### Background Art

It is known that control of voluntary bowel functions can be limited or totally absent in patients having particular disabilities, for instance those patients suffering from bifid spine, consequences of spinal marrow injuries, or multiple sclerosis. Those patients have little or no ability to sense the presence of faeces in the colon terminal part and the rectum, to feel the stimulus to evacuate and to attend to the evacuation functions in self-sufficient and natural way.

For these people a proper system for controlling the bowel functions is of fundamental importance, as it allows them to manage social relationships quite safely and, all in all, to improve the quality of their life.

In this regard, an important aid is given by the colon irrigation, performed transanally. The basic technique of this operation provides that a suitable quantity of water at a temperature similar to that of the body is introduced into the colon by means of a catheter, with timescale such as to avoid distention of the colon or the lower rectum, and therefore not annoying the patient. The presence of water induces peristaltic movements in the colon, which tend to push the faeces toward the rectum, and moisturizes the faeces at the same time causing a noticeable softening thereof.

After a predetermined period of time, usually a few minutes, the water and the faeces are expelled through the sphincters.

To effect the irrigation operations simple devices have been widely used, even in hospitals, which consist of a bag filled with water, connected to a catheter by means of a hose. The bag is supported at a suitable height with respect to the user, so that the water can flow in the colon of the same by the action of gravity.

The above described devices are simple to manufacture and operate, even if they have several drawbacks. First, in fact, to operate properly the bag should be filled, and then positioned at a precise height above the user. This requires the intervention of a skilled assistant, especially in the case of a person with limited mobility, and can make seriously complicated the life organization of those who need daily or almost daily repetition of the operation. In addition, during the irrigation operation, early and unwanted water leaks from the bowels can occur.

To overcome the latter drawback, it is known to use an elastic membrane, arranged coaxially onto the catheter near its terminal portion. The catheter is introduced into the rectum until the membrane goes beyond the barrier of the sphincters. The membrane is then inflated until it blocks the anal channel, before the introduction of the irrigation liquid.

A further known irrigation device includes a bag for the water, provided with an opening closable with a snap-on cap, and a catheter. This latter includes the above-mentioned blocking system for the anal channel with the inflatable membrane. The bag is connected to the catheter with interposition of a hand three-way valve, operable by means of a knob rotatable on three operating positions plus an idling one.

Depending on the operating position of the valve, a hand-operated pump joined to the same valve injects air into the inflatable membrane or sends water from the bag to the catheter. In a third position, the valve allows discharging air from inside the inflatable membrane.

With the device described above, both the raised position of the bag and the leaks of liquid during the irrigation operation, are avoided.

Although it is more practical than the previous irrigation systems, the above-mentioned device has, however, some drawbacks. The user, after connecting the catheter to the outlet pipe, should fill the bag with water at the right temperature; then, he must close the bag by means of the snap-on cap, and insert the catheter into the anal channel in the correct position. At this point, the irrigation procedure provides for the positioning of the switching knob in the position of inflation of the elastic membrane, and manual actuation of the pump until it achieves the correct closure of the channel. Subsequently, the switching knob is moved to the water injection position, and an action with a certain force is performed on the pump and with a determined pumping frequency (a frequency of about one action per second is suggested, to avoid discomfort to the rectum) to introduce the correct amount of water in the colon.

After a specified period of time, which must be timed by the person performing the operation, the switching knob must be moved to the pressure discharging position, to deflate the elastic membrane. The catheter can then be removed and the irrigation operation comes to an end in the traditional manner, by emptying the bowels in natural or assisted way.

The valve must then be moved to the idling position, and the catheter used is removed from the device.

It appears that the use of the irrigation device as described above involves the execution of a series of manual operations, some of which require application of a certain force. Such operations may be also very complicated and difficult, if not impossible, to carry out by a user with limited movement coordination, or with brain dysfunction that anyway influences the ability to perform in the proper sequence a plurality of prearranged movements.

A user with movement or sensory handicaps must essentially be able to act on the pump to inflate the elastic membrane, and recognize the correct inflating pressure, something certainly not easy for those who have problems of sensitivity and response to stimuli in the relevant area. He or she must also act repeatedly on the switching valve, in the correct sequence. Finally, he or she must also act on the pump to transfer the correct amount of water from the bag to his or her colon. Since this quantity can greatly exceed half liter, the number of pumping operations can also be high and its execution is time-consuming.

Another drawback lies in the fact that the components of the irrigation device described above must be replaced with a certain frequency. In particular, the catheter is certainly disposable, and must be replaced after each use. The bag should be replaced every fifteen days, especially if the irrigations are made daily, in order to prevent leakages of water due to failure by mechanical stress. The control unit, i.e. the switching valve and the relative pump, must be replaced at least every six months, but preferably with greater frequency.

In the long run, costs that a person faces due to the use of said irrigation device can become quite high, and discourage adoption by a substantial percentage of potential users.

From the WO 01/08614A1, filed in the name of Zassi Medical Evolution Inc., Von Dyck Peter M. and Schneider JA, an apparatus is known for the irrigation of ostomized patients, including a reservoir for the irrigation fluid disposed within a rigid container, a pump also disposed within the container and adapted to convey the liquid in the user bowels, and an operation sequence control device. The invention described therein, however, relates to an operation entirely different from a transanal irrigation, does not address the issues arising from an operation performed through a sphincter, nor provide means to address them.

### Disclosure of Invention

### Technical Problem

An object of the present invention is therefore to provide a device for transanal irrigation capable of freeing the user, and particularly the user with motion, sensory or coordination handicaps, from the execution of relatively complex, repetitive, tiring operations, or that require the same to identify the achievement of optimal situations in the course of the irrigation operation.

Another object of the invention is to provide a device capable of recognizing autonomously the attainment of the aforementioned optimal operating situations.

A further object of the invention is to provide a device capable of performing in a repeatable and reliable way all preparation and execution steps of the irrigation, in a manner substantially independent of the movement control ability of the user.

### Technical Solution

The above-mentioned objects are all achieved, in accordance with the content of the claims, by an apparatus for carrying out transanal irrigations which includes a reservoir for containing the irrigation water, a catheter set in fluid communication with the reservoir by means of a first conduit, and an elastic membrane aimed at obstructing the anal channel of the user, associated with the catheter and connected to a second conduit.

The apparatus further comprises a first electropump interposed between the catheter and the reservoir; a second electropump, designed to pump air into the membrane to block the anal channel of the user; a computerized control unit, designed to receive commands for carrying out the irrigation from the user and consequently to enable activation means of said electropumps first and second and a solenoid valve, designed to block the second conduit. The above enablement is made in accordance with timings defined by configuration parameters stored in a data memory area of the control unit.

### Advantageous Effects

The apparatus for transanal irrigation according to the invention and as described above allows significant advantages in respect of the conventional irrigation devices. First, it allows to release the user, and particularly the user with motion, sensory or coordination handicaps, from the execution of relatively complex, repetitive, tiring operations, or that otherwise require coordination of movements. It is also not necessary that the user effects specific evaluations about the achievement of optimal, or at least acceptable, situations in the course of the irrigation operation.

A further advantage provided by the invention allows obtaining to perform all operations for preparing and carrying out the operation in a repeatable and reliable way, by using stored and constant configuration parameters, in a way completely independent from the intervention ability of the user.

Still another advantage of the invention is to allow a specialist to acquire, store, modify and reconfigure all the significant irrigation operation parameters for all his/her patients in need of such apparatus.

### Description of Drawings

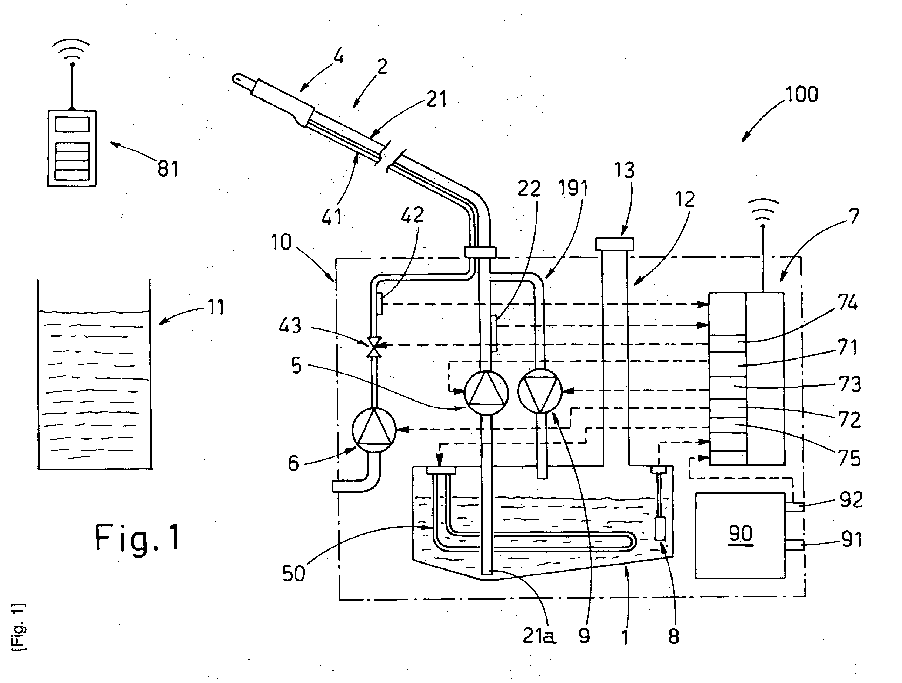

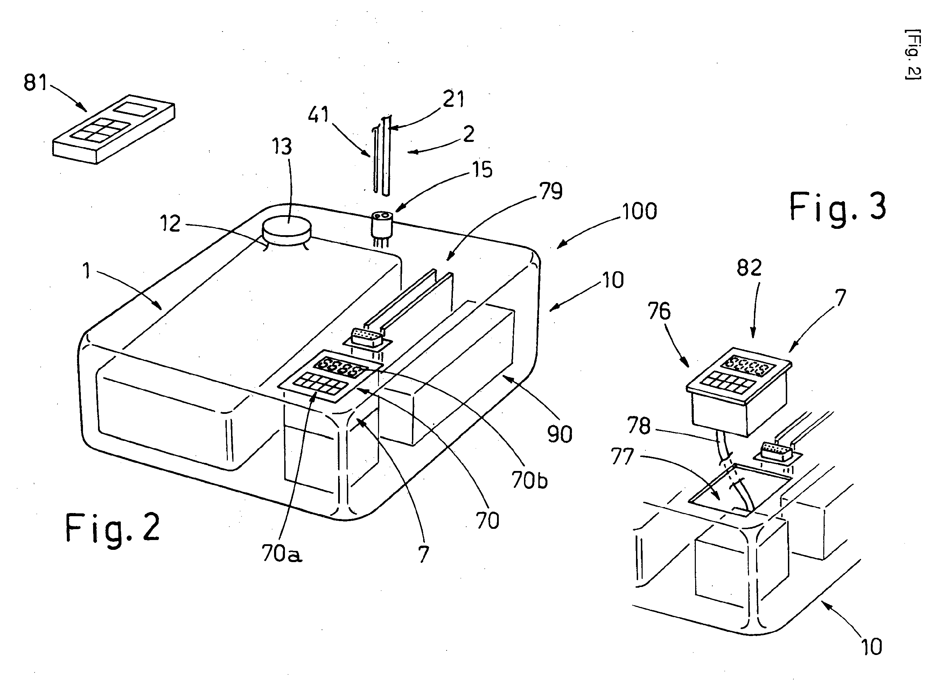

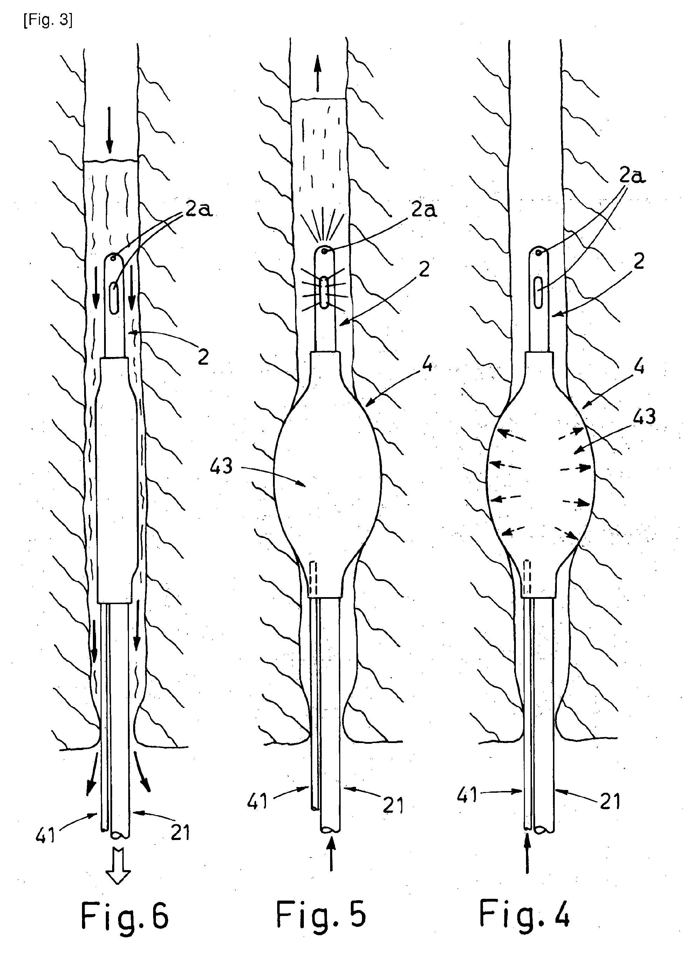

The characteristics of the invention, as they will result from the claims, are pointed out in the following detailed description, with reference to the enclosed drawings, in which:
1. - Figure 1 shows a functional blocks diagrammatic view of an irrigation device made in accordance with a first embodiment of the present invention;
1. - Figure 2 shows a transparency perspective view of a possible embodiment of the irrigation device in accordance with the invention;
1. - Figure 3 shows a detail of the view in Figure 2 of a second embodiment of the invention;
1. - Figure 4 shows a schematic sectional view of the catheter terminal portion in a first operation step;
1. - Figure 5 shows the catheter portion of Figure 4 in a subsequent operation step;
1. - Figure 6 shows the catheter portion of Figure 5 in a further subsequent operation step.

### Best Mode

With reference to Figures 1 and 2, reference numeral 100 denotes, as a whole, an apparatus for carrying out transanal irrigations, made in accordance with a first but not exclusive embodiment of the invention.

In particular, apparatus 100 includes a rigid container 10, having such dimensions as to be easily handled and transported by a user. Inside the container 10, there is housed and secured a reservoir 1, designed to contain water, or any other liquid suitable for making intestinal irrigations, in an amount sufficient to meet the normal requirements of the above-mentioned operation.

The reservoir 1 includes, in its upper part, a collection opening 12, leading to the outside of the container 10 and provided with a suitable cap 13. One end 21a of a first conduit 21 dips into the bottom portion of the reservoir 1, and goes out from the same reservoir 1, from the container 10 and is connected, at the opposite end 21b, to a catheter 2, to set this latter in fluid communication with the above-mentioned reservoir 1. The catheter 2 features, at its distal end, one or more openings 2a, designed to set the channel of the catheter 2 in communication with the outside.

In the practical realization, the first conduit 21 is split, and the part of it that is inside the container 10 opens into a plug of a two-way connector 15, the structure of which will be better described in detail in the following. The portion of the first conduit 21 outside the container 10 is removably connected to the connector 15.

Obstruction means 4 for the obstruction of the anal channel of the user of apparatus 100 are associated, in known manner, to the catheter 2. Such obstruction means 4 are of the type comprising an elastic, substantially cylindrical, membrane 43, inflatable and fastened to the outside of the catheter 2 (see also Figures 4 to 6) and in coaxial relation with the same, upstream of the openings 2a.

The inside of the elastic membrane 43 is set in fluid communication with a second conduit 41, designed to supply air under pressure to the same elastic membrane 43, which extends parallel to the first conduit 21 and is preferably fastened to the side wall thereof. The manner of securing the two conduits are fully known, and may include simply gluing the mating walls, or incorporating the two conduits in a block of plastic material that makes them essentially a single body.

The second conduit 41 ends inside the container 10 and is also split. The conduit portion inside the container 10 leads to a second plug of the previously mentioned connector 15, set beside the corresponding outlet of the first conduit 21. The outer portion of the second conduit 41 is connected detachably, together with the corresponding outer portion of the first conduit 21, to the same connector 15. The latter can be any commercially available quick coupling model, which matches with the dimensions of the conduits and capable of achieving a fluid-tight connection.

In this way, the assembly including the catheter 2, the elastic membrane 4 and the outer portions of the conduits first 21 and second 41 is completely and easily removable from the rest of the apparatus 100, and can be replaced after each use by the same user.

The apparatus 100 further comprises a first electropump 5, secured inside the container 10 and mounted in series with said first conduit 21, so as to be interposed between the catheter and the reservoir 1. The first electropump 5 is intended to draw water from the latter during the execution of the transanal irrigation operation and to send it to the catheter 2, for conveying it into the colon rectal portion of the user.

A second electropump 6 is also mounted in series to the inner part of the second conduit 41, and is set in fluid communication with the same conduit and with the external environment. The second electropump 6 is intended to draw air from the external environment, after insertion of the catheter into the user rectum, and to send it under pressure to the elastic membrane 4, to inflate it and obstruct the anal channel.

Downstream of the second electropump 6, Flow cut off means 43, preferably constituted by a solenoid valve for use in pneumatics, are provided along the second conduit 41, which can be activated on command capable of interrupting the flow of air along the same conduit 41, to maintain a correct pressure inside the elastic membrane 4 once inflated.

According to the first embodiment of the invention, the apparatus 100 also provides a third electropump 9, housed inside the container 10 and set in fluid communication with the catheter 2 and the reservoir 1 by means of a third conduit 91. The latter branches off from the inner portion of the first conduit 21 and ends, downstream of the third electropump 9, inside the reservoir 1.

The third electropump 9 is intended to draw water to perform the irrigation from an external source 11, through the catheter 2, and to convey it to the reservoir 1 on command imparted by the user, as will become clearer in the following.

Of course, various embodiments of the pneumatic-hydraulic configuration of the apparatus 100 so far described and illustrated in Figure 1, are possible to imagine. For example, the electropumps first 5 and third 9 may be replaced by a reversible action electropump, intended to be activated for pumping water to or from the reservoir 1, in accordance with the current step of the irrigation operation.

In a further different embodiment the first electropump 1 and the second electropump 2 are replaced with a single electropump, and that the same is alternately connected to the first and second conduits by means of relative pneumo-hydraulic electrically activated switching circuits, easy to implement and, therefore, which will not be further discussed.

The apparatus 100 also includes a computerized control unit 7, essentially constituted by an industrial microcontroller having adequate performance (see Figure 2), provided with a user interface 70 including a keypad 70th as input, and a display 70b as output, and also provided with a suitable number of input/output ports. The control unit 7 also comprises an rewritable permanent memory area, intended to store a plurality of irrigation operation configuration parameters and a program for management of the same.

A plurality of activation means 71,72,73,74 are also associated with the control unit 7 for operation of said electropumps first 5, second 6 and third 9, and the solenoid valve 43 for the air flow cut off in the second conduit 41, respectively. The above mentioned activation means consist of, for example, contactors having suitable electrical characteristics, connected to respective input/output ports of the control unit 7. In Figure 2, the activation means are housed on printed circuit boards 79.

Further input/output ports connect the control unit 7 to pressure sensors first 22 and second 42, associated to the aforesaid conduits first 21 and second 41, respectively. These sensors are designed to detect the water pressure in the first conduit 21 and the air pressure in the second conduit 41, respectively, to provide feedback information to the irrigation operation management program, as will be apparent from the following functional description of the apparatus 100.

Inside the reservoir 1, there is further provided a temperature sensor 8, electrically connected to a signal input (input/output port) of the control unit 7 for detecting the temperature of the water present inside the reservoir 1, transmitting the information to the irrigation operation management program, and allowing the same to enable/disable operations depending on the water temperature being or not within a predetermined acceptable range.

In the illustrated embodiment, the apparatus 100 includes a heater 50, which is also activated on command by said control unit 7 by relative activation means 75, and intended to heat the water introduced into the reservoir 1 up to a temperature within the above mentioned predetermined acceptable range. The heater 50 consists essentially of a power resistor, of tubular type, inserted inside the reservoir 1 (shown as an example in Figure 1) or of the type with a heating plate, positioned at the bottom of the same reservoir, inside or outside the same.

The apparatus 100 also includes a power supply group 90, consisting of an accumulator battery and a corresponding low voltage conversion device for recharging from the network, of known construction. The conversion and recharging device is connected to the network by means of a socket 91, to which a network cable, not shown, is connected.

Safety enabling means 92 are also provided in correspondence of the above mentioned socket 91, which are schematically shown in Figure 1 and consist of a micro-switch electrically connected to a corresponding input/output port of the control unit 7 and which are designed to enable the execution of the irrigation operations, as described in the functional section of the present description.

In order to make the use of the apparatus 100 easier by the user having movement handicaps, remote control means 81, 82, are also provided, which allow the activation and the remote control of the irrigation operating sequence.

In the first illustrated embodiment, such remote control means 81 include a remote control, commonly supplied as an accessory of industrial microcontroller models which constitute the control unit 7, for example of the type operating in the infrared or radio band.

The remote control 81 is provided with a plurality of keys, the pressure exerted on each of which corresponds to as many commands to impart to the irrigation operation management program resident in the control unit 7.

### Mode for Invention

According to a second embodiment of the invention, which is schematically shown in Figure 3, remote control means 82 are provided, which include the entire control unit 7 or part thereof. In this case, the control unit is normally housed in a compartment 77 provided in the rigid container 10 and is removable therefrom for carrying out the irrigation control operations.

The control unit is connected to the other devices of the apparatus 100 by means of a multi-cable 78, which is also removable and housed in the compartment 77. The length of this cable is such as to allow the user to control the execution of operations while being seated, while the apparatus is on the floor or in any other suitable position.

The irrigation operation management program includes the performance of the command and control procedures which may be different, depending on the degree of automation which is considered suitable for the apparatus. In particular, the procedure can be controlled by the user, step by step, by pressing keys suitably provided on the remote control 81 or on the removable control unit 82. Alternatively, the user can operate the entire step sequence with a single command. In this case, the execution timing of the different steps can be stored during the setting of the custom configuration for the particular user of the apparatus 100.

The configuration setting can be done directly by means of the user interface of the control unit 7. In general, for each user, this is done by the medical practitioner who takes care of the user, on the basis of empirically obtained results, and is usually only occasionally changed.

In order to facilitate the task of the said medical practitioner, the apparatus 100 can be equipped with a module for communication with an external processing unit, resident in the control unit 7, and a configuration program, resident and operating in the external processing unit. The communication module, consisting of blocks of computer program and standard communication interfaces, is made with known methods and is capable of acquiring and displaying the configuration parameters present in the data area of the control unit 7 and allows the specialist to edit, transfer and install them in the same data area.

The operation of the equipment for transanali irrigation 100 will be hereinafter described with reference to an exemplary irrigation session in which a user has an apparatus 100 already configured on the basis of his/her needs, and therefore ready for use, provided with a remote control 81.

The first operation that the user has to perform is to prepare the catheter 2, supplied in a package and with its own connection tube of suitable length. The catheter 2 is preferably with a lubricant film already active, so it is not usually necessary to activate it in advance by introducing water into the package. Therefore, it is sufficient to remove the catheter 2 from the package and connect the end of the connecting hose to the connector 15, by means of a snap-on or screw joint.

At this point the user has two alternatives. The first one is to remove the cap 13 from the collection opening 12 and to fill the reservoir 1 with a quantity of water at least equal to that necessary for the irrigation. The second one is to simply dip the catheter 2 in an available vessel 11, for example a bottle or even the tub of the sink, containing a sufficient quantity of water, and then press a particular key on the remote control 81, designed to start the loading operation of the reservoir 1.

Based on information on the quantity of the irrigation water stored in the data area of the control unit 7, the management program provides for activation of the third electropump 9 for a predefined period of time, calculated on the basis of this information and the flow rate characteristics of the electropump 9 model, thereby loading the reservoir 1 with a quantity at least equal to that necessary.

A further pressing on a special key on the remote control 81 then transfers the control of the operations to the thermoregulation management procedure for the water in the reservoir 1. This procedure provides firstly to detect an information concerning the current water temperature in the reservoir 1, by the sensor 8. If the temperature is too high, the sequence is stopped, and a 'very hot water' signal is displayed on the display 70b of the control unit 7. If, instead, the temperature is below the expected range, the program controls the activation of the heater 50, until the temperature sensor 8 detects the attainment of an acceptable temperature. The achieving of this operation state is also displayed on the display 70b.

The foregoing operations can be performed with the apparatus 100 connected to the electric main. The micro-switch 92 detects this condition and provides the information to the control unit 7, which, anyway, allows execution thereof. Subsequent operations must instead be carried out with low voltage power, for safety reasons, supplied by the battery of accumulators. The user is therefore required to disconnect the power cord from the main socket 91. This operation is detected by the micro-switch 92 and, through this, by the control unit 7, which enables the continuation of the irrigation operational steps.

At this point, the user shall introduce the catheter 2 in the rectum, in a position such that the elastic membrane 4 is positioned immediately beyond the sphincter. Then he can operate the actual irrigation sequence, in automatic mode, by pressing a proper key on the remote control 81.

This last event causes the activation of the main irrigation sequence by the management program, which starts by opening the solenoid valve 43 and the second electropump 6, which supplies air under pressure to the elastic membrane 4, making it inflate. After a predetermined period of time or when the second pressure sensor 42, if present, has detected a sufficient inflation pressure, the program commands the closing of the solenoid valve 43.

After a further period of time, necessary for settling the membrane 4 and refining the closure of the anal channel, the program controls the activation of the first electropump 5, which starts introducing water into the rectum of the user through the catheter 2. The first pressure sensor 22, if present, provides an indication so that, in this case, a supply pressure considered optimal for a correct inflow of water in the colon is not exceeded. In correspondence, the program may command temporary stops of the first electropump 5, so as to stabilize the flow of water.

After an activation predetermined period of time, reckoned on the basis of the quantity of irrigation water to be provided and the flow rate of the first electropump 5, this latter is deactivated. In this step, the presence of the inflated elastic membrane 4 prevents any outflow of water from the sphincter of the user, regardless of the control capabilities of the same.

When the permanence time of the irrigation water in the colon is considered concluded, in accordance with the relative time parameter appropriately stored in the data area of the control unit 7, the management program commands the opening of the solenoid valve 43, which allows the air present in the elastic membrane 4 to flow to the external environment, and the latter to deflate. The main irrigation sequence is at this point completed.

The user can then pull the catheter 2 out from the rectum and, optionally, operate the execution of a further emptying step of the reservoir 1, by pressing a proper key on the remote control 81. Finally, he shall remove the catheter 2 and its connecting tube 15 from the connector and throw it away. The evacuation functions of water and contents of the colon can then take place in the normal manner.

The apparatus 100 is then immediately ready for a new operating cycle. Alternatively, a semi-automatic mode of operation can be selected, or factory-set according to requirements. With such operation mode, the user will provide manually the activation commands of the reservoir 1 filling step, water heating, elastic membrane 4 inflation, introduction of irrigation water in the colon, and discharge of the air from the same membrane. During these steps, the program can of course provide help for the user as regards, for example, the calculation of pause times, etc.

It is understood that what has been described above by way of mere examples and is not limiting. Therefore, possible modifications and variations of the invention are to be considered comprised within the scope of the present technical solution, as described above and claimed hereinafter.

## Claims

1. An apparatus for performing transanal irrigations, including a reservoir 1 for containing the irrigation water or other liquid for irrigation, a catheter 2 set in fluid communication with the reservoir through a first conduit 21, for introducing said water into the bowel end portion of a user, and obstruction means 4 for obstructing the anal channel of said user, associated with the catheter 2 and connected to a second conduit 41, first pumping means 5, operated on command and interposed between said catheter 2 and said reservoir 1 to draw water from this latter and to convey it to said catheter 2; said apparatus 100 being **characterized by** further including: second pumping means 6; operated on command and set in fluid communication with said second conduit 41 and with the external environment, designed to pump air into said obstruction means 4 for obstruction of said anal channel, and to release air in the external environment to allow extraction of said catheter 2; a computerized control unit 7, designed to receive commands for performing the irrigation from said user and consequently to enable activation means 71, 72, 75 of said first 5 and second 6 pumping means as well as flow cut off means 43, located downstream of said second pumping means 6 and designed to block said second conduit 41, the above enablement being made in accordance with timings defined by configuration parameters stored in a data memory area of the same control unit 7; a temperature sensor 8, situated in said reservoir 1, electrically connected to a corresponding signal input of said control unit 7 for detecting the temperature of the water present inside the reservoir 1, to allow the same control unit 7 to enable or disable the irrigation operating sequence; a rigid container 10, adapted to house at least said reservoir 1, first 5 and second 6 pumping means and control unit 7; remote control means 81,82, designed to allow said user to remotely activate said irrigation operational sequence; a software procedure for handling said irrigation operational sequence, operable in said control unit 7 and fit to receive and interpret said user imparted commands, to activate at least said first 5 and second 6 pumping means, based on information provided by said temperature sensor 8.

2. An apparatus according to claim 1, **characterized by** further including third pumping means 9, consisting of a third electropump, set in fluid communication with said catheter 2 and said reservoir 1 via a third conduit 91, operable on command by said control unit 7 by means of corresponding activation means 73, intended to draw water for irrigation from an external source 11 through the catheter 2 and to convey it to said reservoir 1 in accordance with timings defined by corresponding configuration parameters stored in the data memory of the control unit 7.

3. An apparatus according to claim 1, **characterised in that** said first 5 and second 6 pumping means comprise a single electropump, having its inlet and its outlet connected to said conduits first 21 and second 41 by means of a pneumatic-hydraulic switching circuit, which can be operated by said control unit 7.

4. An apparatus according to claim 2, **characterised in that** said first 5 and third 9 pumping means comprise a single bi-directional pumping device, the pumping direction of the same being defined by said control unit 7 in accordance with the operative step of irrigation.

5. An apparatus according to claim 1, **characterised in that** said remote control means 81 consist of a remote control.

6. An apparatus according to claim 1, **characterised in that** said remote control means 82 include the entire control unit 7 or part thereof, housed in a compartment 77 provided in said rigid container 10 and removable therefrom, set in data and signal communication with the remaining devices of the apparatus 100 by means of at least one multi-cable 78 of suitable length.

7. An apparatus according to claim 1, **characterized by** further including a first 22 and a second 42 pressure sensor, associated with said conduits first 21 and second 22 respectively and electrically connected to corresponding signal inputs of said control unit 7, designed to detect the water pressure in said first conduit 21 and the air pressure in said second conduit 41 respectively.

8. An apparatus according to claim 1, **characterized by** further including a heater 50, placed inside or near said reservoir 1 and operable on command by corresponding activation means 74 provided in said control unit 7.

9. An apparatus according to claim 1, claim 2 or claim 8, **characterised in that** a computer program is resident in said control unit 7, which program is designed to receive instructions from the user either locally or through said remote control means 75, 76 to implement an irrigation step operational sequence, with timing defined by said stored configuration parameters, including activation and deactivation in a sequence of said activation means 71, 72, 73, 74, 75.

10. An apparatus according to claim 1 or claim 9, **characterised by** including a communication module for communicating with an external processing unit, residing in said control unit 7, and a configuration program, running on said external processing unit, aimed at acquiring said configuration parameters from said control unit 7, modifying and transferring them to the data memory area of said control unit 7.

11. An apparatus according to claim 1, **characterized by** further including safety enabling means 92, arranged at a power main socket 91 and fit to provide said control unit 7 with information relating to the connection status of said power main socket 91 to a corresponding main cable.

## Patentansprüche

1. Eine Vorrichtung zur Durchführung von transanalen Spülungen mit einem Vorratsbehälter 1 zur Aufnahme des Spülungswassers oder einer anderen Flüssigkeit für die Spülungen; einem Katheter 2, der in Fluidverbindung mit dem Vorratsbehälter durch eine erste Leitung 21 steht, um Wasser in den Darm Endabschnitt eines Benutzers einzuführen; und mit dem Katheter 2 zugeordnet und mit einer zweiten Leitung 41 verbunden Versperrungsmitteln 4, die den Analkanal des Benutzers versperren; eine erste Pumpeinrichtung 5, die auf Befehl betrieben werden und zwischen dem Katheter 2 und dem Vorratsbehälter 1 angeordnet ist, um Wasser aus dem Vorratsbehälter zu entnehmen und das an den Katheter 2 zu fördern; die Vorrichtung 100 ist ferner **gekennzeichnet durch**: eine zweite Pumpeinrichtung 6, die auf Befehl betrieben wird und in Fluidverbindung mit der zweiten Leitung 41 sowie mit der äußeren Umgebung ist, wobei die zweite Pumpeinrichtung Luft in die Versperrungsmittel 4 zum Versperrung des Analkanals pumpt und Luft in der äußeren Umgebung entlasst, um die Extraktion des Katheters 2 zu ermöglichen; eine computerisierte Steuereinheit 7, die Befehle zum Durchführen der Bewässerung vom Benutzer empfangt und somit Aktivierungseinrichtungen 71, 72, 75 der ersten 5 und zweiten 6 Pumpeinrichtung sowie Fluss-Absperrmittel 43, die stromabwärts der zweiten Pumpeinrichtung 6 zum Sperren der zweiten Leitung 41 angeordnet sind, aktiviert, wobei die Aktivierung wird in Übereinstimmung mit Zeitpunkten vorgenommen, die anhand von, in einem Datenspeicherbereich der Steuereinheit 7 gespeicherten Konfigurationsparametern definiert werden; einen Temperatursensor 8, der sich innerhalb des Vorratsbehälters 1 befindet und zum Erfassung der Temperatur des Wasser innerhalb des Vorratsbehälters 1, elektrisch mit einem entsprechenden Signaleingang der Steuereinheit 7 verbunden ist, um die Steuereinheit 7 zum Aktivieren oder Deaktivieren des Arbeitsablaufs der Bewässerung zu ermöglichen; einen starrer Behälter 10, in dem zumindest den Vorratsbehälter 1, die erste 5 und zweite 6 Pumpeinrichtung und die Steuereinheit 7 untergebracht werden; Fernbedienungsmittel 81,82, die es dem Benutzer ermöglichen, den Arbeitsablauf der Bewässerung aus der Ferne zu aktivieren; einen Software-Verfahren zum Übernehmen des Arbeitsablaufs der Bewässerung, der in der Steuereinheit 7 betreibbar ist und die Befehle von dem Benutzer vermittelt empfängt und interpretiert, um zumindest die erste 5 und zweite 6 Pumpeinrichtung zu aktivieren, anhand von Informationen, die durch den Temperatursensor 8 abgegeben werden.

2. Eine Vorrichtung nach Anspruch 1, **dadurch gekennzeichnet, dass** die Vorrichtung besteht aus eine dritte Pumpeinrichtung 9, die aus einer, in Fluidverbindung mit dem Katheter 2 und mit dem Vorratsbehälter 1 über eine dritte Leitung 91 gesetzt dritten Elektropumpe besteht, die auf einen Befehl von der Steuereinheit 7 durch entsprechende Betätigungsmittel 73 betrieben wird, wobei di Elektropumpe Wasser für die Bewässerung von einer externen Quelle 11 entnimmt und durch den Katheter 2 das Wasser in den Vorratsbehälter 1, in Übereinstimmung mit Zeitpunkten, die anhand von, in dem Datenspeicher der Steuereinheit 7 gespeicherten Konfigurationsparametern definiert werden, vermittelt.

3. Eine Vorrichtung nach Anspruch 1, **dadurch gekennzeichnet, dass** die erste 5 und zweite 6 Pumpeinrichtung eine einzige Elektropumpe umfassen, die einen Einlaß und einen Auslaß mit den ersten 21 und zweiten 41 Leitungen, mittels eines, durch die Steuereinheit 7 betrieben pneumatisch-hydraulischen Schaltkreis, verbunden hat.

4. Eine Vorrichtung nach Anspruch 2, **dadurch gekennzeichnet, dass** die erste 5 und dritte 6 Pumpeinrichtung eine einzige bidirektionalen Pumpvorrichtung umfassen, wobei die Pumprichtung der Pumpvorrichtung durch die Steuereinheit 7 in Übereinstimmung mit dem Arbeitsablauf der Bewässerung definiert wird.

5. Eine Vorrichtung nach Anspruch 1, **dadurch gekennzeichnet, dass** die Fernbedienungsmittel 81 aus einer Fernsteuerung bestehen.

6. Eine Vorrichtung nach Anspruch 1, **dadurch gekennzeichnet, dass** die Fernbedienungsmittel 82 aus der gesamte, in einer Tasche 77 des starren Behälters 10 untergebracht und davon abnehmbar Steuereinheit 7 bestehen, wobei Steuereinheit in Daten- und Signalkommunikation mit den restlichen Einrichtungen der Vorrichtung 100, mittels zumindest eines Multikabels 78 von geeigneter Länge eingestellt ist.

7. Eine Vorrichtung nach Anspruch 1, weiter **gekennzeichnet durch** eine erste 22 und eine zweite 42 Drucksensor, die mit den ersten 21 und zweiten 22 Leitungen zugeordnet und elektrisch mit den entsprechenden Signaleingängen der Steuerungseinheit 7 verbunden sind, und die den Wasserdruck in der ersten Leitung 21 und der Luftdruck in der zweiten Leitung 41 erfassen.

8. Eine Vorrichtung nach Anspruch 1, weiter **gekennzeichnet durch** eine Heizeinrichtung 50, die innerhalb oder in der Nähe des Vorratsbehälters 1 platziert und auf Befehl **durch** entsprechende in der Steuereinheit 7 vorgesehen Aktivierungsmittel 74 betrieben wird.

9. Eine Vorrichtung nach Anspruch 1, Anspruch 2 oder Anspruch 8, **dadurch gekennzeichnet, dass** die Steuereinheit 7 ein Computerprogramm umfasst, das Anweisungen von dem Benutzer entweder lokal oder über die Fernbedienungsmittel 75, 76 empfängt, um einen Arbeitsablauf der Bewässerung in Übereinstimmung mit anhand von dem gespeicherten Konfigurationsparametern definiert Zeitpunkten, die die Aktivierung und Deaktivierung in einer Sequenz der Aktivierungsmittel 71, 72, 73, 74, 75 beinhalten, umzusetzen.

10. Eine Vorrichtung nach Anspruch 1, oder Anspruch 9, **gekennzeichnet durch** ein in Verbindung mit einer externen und in der Steuereinheit 7 enthaltenen Verarbeitungseinheit Kommunikationsmodul, und ein auf der externen Verarbeitungseinheit laufenden Konfigurationsprogramm, das die Konfigurationsparameter von der Steuereinheit 7 übernimmt um die Parameter zu modifizieren und zu dem Datenspeicherbereich der Steuereinheit 7 zu übertragen.

11. Eine Vorrichtung nach Anspruch 1, weiter **gekennzeichnet durch** Sicherheitsfreigabemittel 92, die in einem Hauptstrombuchse 91 angeordnet sind und der Steuereinheit 7 Informationen auf den Status der Verbindung des Hauptstrombuchse 91 an einem entsprechenden Hauptkabel bereitstellt.

## Revendications

1. Appareil pour réaliser des irrigations transanales, comprenant un réservoir 1 destiné à contenir de l'eau d'irrigation ou d'un autre liquide pour l'irrigation, un cathéter 2 en communication fluidique avec le réservoir par l'intermédiaire d'un premier conduit 21, pour introduire ladite eau dans la partie d'extrémité de l'intestin d'un utilisateur, et des moyens d'obstruction 4 pour boucher le canal anal dudit utilisateur, lesdits moyens d'obstruction étant associés au cathéter 2 et reliés à un second conduit 41, des premiers moyens de pompage 5, fonctionnant sur commande et interposé entre ledit cathéter 2 et ledit réservoir 1 pour puiser l'eau du réservoir et pour le transporter audit cathéter 2; ledit appareil 100 étant en outre caractérisé en ce qu'il comporte en outre: des deuxièmes moyens de pompage 6, fonctionnant sur commande et aménagé en communication fluidique avec ledit second conduit 41 et avec l'environnement extérieur, destinés à pomper de l'air dans ladite moyens d'obstruction 4 pour l'obstruction du canal anal, et pour libérer l'air dans l'environnement extérieur pour permettre l'extraction dudit cathéter 2; une unité d'ordinateur de commande 7, conçu pour recevoir des commandes de l'utilisateur pour effectuer l'irrigation et par conséquent pour actionner les moyens d'activation 71, 72, 75 desdits premier 5 et second 6 moyens de pompage ainsi que moyen de coupure du flux 43, située en aval de lesdits deuxièmes moyens de pompage 6 et conçu pour bloquer ledit second conduit 41, l'actionnement étant faite conformément avec les temporisations définis par des paramètres de configuration stockées dans une zone de mémoire de données de la même unité de commande 7; un capteur de température 8, situé dans ledit réservoir 1, relié électriquement à une entrée du signal correspondant de ladite unité de commande 7 pour détecter la température de l'eau présente à l'intérieur du réservoir 1, pour permettre à la même unité de commande 7 pour activer ou désactiver le séquence de fonctionnement de l'irrigation; un récipient rigide 10, pour loger au moins ledit réservoir 1, les premiers 5 et les deuxièmes 6 moyens de pompage et l'unité de commande 7; des moyens de commande à distance 81, 82, conçu pour permettre audit utilisateur d'activer à distance ladite séquence de fonctionnement de l'irrigation; un programme informatique pour la gestion de la séquence de fonctionnement de l'irrigation, qui peut être actionné dans ladite unité de commande 7 et apte à recevoir et interpréter les commandes impartie par ledit utilisateur, pour activer au moins ledit premiers 5 et deuxièmes 6 moyens de pompage, sur la base de l'information fournie par ledit capteur de température 8.

2. Appareil selon la revendication 1, **caractérisé en ce qu'**il comprend en outre: troisièmes moyens de pompage 9, consistant en une troisième électropompe, en communication fluidique avec ledit cathéter 2 et ledit réservoir 1 par l'intermédiaire d'un troisième conduit 1, qui pouvant être actionné sur commande par ladite unité de commande 7 par l'intermédiaire des moyens d'activation 73 correspondant, destiné à puiser de l'eau pour l'irrigation d'une source externe 11 par l'intermédiaire du cathéter 2 et pour le transporter audit réservoir 1; conformément avec les temporisations définis par des paramètres de configuration correspondants stockées dans une mémoire de données de liunité de commande 7.

3. Appareil selon la revendication 1, **caractérisé en ce qu'**il lesdits premiers 5 et deuxièmes 6 moyens de pompage comprennent une seule électropompe, ayant son entrée et sa sortie connectée à lesdits conduits première 21 et deuxième 41 par l'intermédiaire d'un circuit de commutation pneumatique-hydraulique, qui peut être actionné par ladite unité de commande 7.

4. Appareil selon la revendication 2, **caractérisé en ce qu'**il lesdits premiers 5 et troisièmes 9 moyens de pompage comprennent un unique dispositif de pompage bi-directionnel, la direction de pompage de le même étant défini par ladite unité de commande 7, conformément à l'étape de fonctionnement de l'irrigation.

5. Appareil selon la revendication 1, **caractérisé en ce que** lesdites moyens de commande à distance 81 sont constitués d'une télécommande.

6. Appareil selon la revendication 1, **caractérisé en ce que** lesdites moyens de commande à distance 82 comprennent toute l'unité de commande 7 ou partie de celui-ci, logé dans un compartiment 77 prévu dans ledit récipient rigide 10 et amovible de celle-ci, et en communication de données et de signal avec les dispositifs restants de l'appareil 100 par l'intermédiaire d'au moins une multi-câble 78 de longueur appropriée.

7. Appareil selon la revendication 1, **caractérisé en ce qu'**il comprend en outre_un premier 22 et un deuxième 42 capteur de pression, qui sont associé à ladite premier 21 et deuxième 22 conduits respectivement, et qui sont connectés électriquement à des entrées de signaux correspondant de ladite unité de commande 7, conçu pour détecter la pression de l'eau dans ledit premier conduit 21 et la pression d'air dans ledit deuxième conduit 41 respectivement.

8. Appareil selon la revendication 1, **caractérisé en ce qu'**il comprend en outre un dispositif de chauffage 50, placé à l'intérieur ou à proximité dudit réservoir 1 et qu'il pout être actionné sur une commande par l'activation des moyens 74 correspondant prévu dans ladite unité de commande 7.

9. Appareil selon la revendication 1, la revendication 2 ou la revendication 8, **caractérisé en ce que** un programme informatique est résident dans ladite unité de commande 7, lequel programme est conçu pour recevoir des instructions provenant de l'utilisateur, soit localement, soit par ledit moyen de commande à distance 75, 76 pour mettre en oeuvre une séquence de l'étape de fonctionnement d'irrigation, avec les temporisations définis par lesdits paramètres de configuration, la séquence comprenant l'activation et la désactivation d'une séquence mémorisée d'activation desdits moyens 71, 72, 73, 74, 75.

10. Appareil selon la revendication 1 ou la revendication 9, **caractérisé en ce qu'**il comprend un module de communication avec une unité de traitement externe et contenue dans ladite unité de commande 7, et un programme de configuration exécuté sur l'unité de traitement externe, en vue de l'acquisition desdits paramètres de configuration à partir de ladite unité de commande 7, de les modification et de les transférer vers la zone de mémoire de données de ladite unité de commande 7.

11. Appareil selon la revendication 1, **caractérisé en ce qu'**il comprend en outre moyens d'activation sécurité 92, disposés à une prise de courant d'alimentation principal 91 et apte à fournir ladite unité de commande 7 des informations relatives à l'état de connexion de ladite prise de courant d'alimentation principal 91 à un câble principal correspondant.
